# EUROPÄISCHE PATENTSCHRIFT

(11) **EP 0 809 625 B1**
(45) Veröffentlichungstag und Bekanntmachung des Hinweises auf die Patenterteilung: **11.04.2001**
(21) Anmeldenummer: 95940196.9
(22) Anmeldetag: 17.11.1995
(51) Int. Cl.: C07C 233/01, A61K 7/00, C11D 1/66, B01F 17/00

(54) **ANIONISCHE AMPHIPHILE VERBINDUNGEN MIT MINDESTENS ZWEI HYDROPHILEN UND MINDESTENS ZWEI HYDROPHOBEN GRUPPEN AUF DER BASIS VON DICARBONSÄUREDIAMIDEN**
ANIONIC AMPHIPHILIC COMPOUNDS WITH AT LEAST TWO HYDROPHILIC AND AT LEAST TWO HYDROPHOBIC GROUPS BASED ON DICARBOXYLIC ACID DIAMIDES
COMPOSES AMPHIPHILES ANIONIQUES, COMPORTANT AU MOINS DEUX GROUPEMENTS HYDROPHILES ET AU MOINS DEUX GROUPEMENTS HYDROPHOBES, A BASE DE DIAMIDES D'ACIDES DICARBOXYLIQUES

(30) Priorität: 17.02.1995 DE 19505368
(43) Veröffentlichungstag der Anmeldung: 03.12.1997
(73) Patentinhaber: RWE-DEA Aktiengesellschaft für Mineraloel und Chemie, 22297 Hamburg (DE)
(72) Erfinder: KWETKAT, Klaus, D-44534 Lünen (DE); KOCH, Herbert, D-46282 Dorsten (DE); RUBACK, Wulf, D-48249 Dülmen (DE)
(74) Vertreter: Schupfner, Gerhard D., Dr. Dipl.-Chem.
(86) Internationale Anmeldenummer: EP9504530
(87) Internationale Veröffentlichungsnummer: WO9625388

(56) Entgegenhaltungen:
- US-A- 2 968 675
- US-A- 5 160 450
- PATENT ABSTRACTS OF JAPAN vol. 016 no. 383 (C-0974) ,17.August 1992 & JP,A,04 124165 (KANEBO LTD) 24.April 1992, in der Anmeldung erwähnt
- PATENT ABSTRACTS OF JAPAN vol. 014 no. 090 (C-0691) ,20.Februar 1990 & JP,A,01 304033 (LION CORP) 7.Dezember 1989, in der Anmeldung erwähnt
- JAOCS, Bd. 65, Nr. 5, 1988 Seiten 820-825, T.J. MICICH , W.M. LINFIELD 'wetting properties of nonionics from branched fatty diamides'
- MAKROMOL. CHEM., Bd. 180, 1979 Seiten 929-937, E. GÜNSTER AND R.C. SCHULZ 'Polarographische Untersuchungen an monomeren und polymeren N-Chlorcarbonamiden'
- MAKROMOL. CHEM., Bd. 177, 1976 Seiten 3441-3446, H. YAMAGUCHI UND R.C. SCHULZ 'Chlorierung eines optisch aktiven Polyamids'

## Beschreibung

Die Erfindung betrifft amphiphile Verbindungen mit mindestens zwei hydrophilen und mindestens zwei hydrophoben Gruppen auf der Basis von Diamiden.

Als amphiphile Substanzen sind eine große Vielfalt an anionischen, kationischen, nichtionischen und zwitterionischen Verbindungen bekannt. Die weitaus meisten dieser Substanzen bestehen aus einer hydrophilen Kopfgruppe und wenigstens einem hydrophoben Teil.

Bei den amphiphilen Substanzen gibt es aus ökologischen Gründen, z. B. bezüglich der Verringerung des Verpackungs- und Transportaufwandes, die Notwendigkeit, immer größere Wirkung pro Masse an eingesetzter Substanz zu erzielen. Da eine Optimierung durch Mischung von amphiphilen Substanzen nur sehr begrenzt weiterführt, sind neue amphiphile Substanzen mit einem höheren Wirkungsgrad erforderlich. Es müssen daher insbesondere Stoffe mit niedrigeren kritischen Micellbildungskonzentrationen und/oder niedrigeren Ober- und Grenzflächenspannungen gefunden werden, um die Einsatzmengen an Wirksubstanz deutlich reduzieren zu können.

Erste Lösungsansätze in dieser Richtung durch Verdoppelung eines Teils der Struktur (hydrophile Kopfgruppe, hydrophobe Gruppe) sind bereits bekannt. So können kationische grenzflächenaktive Verbindungen durch die Addition von langkettigen Alkylhalogeniden an permethylierte Alkylendiamine erhalten werden [R. Zana, M. Benrraou, R. Rueff, Langmuir, 7 (1991) 1072; R. Zana, Y. Talmon, Nature, 362 (1993) 228; E. Alami, G. Beinert, P. Marie, R. Zana, Langmuir, 9 (1993) 1465].

Anionische grenzflächenaktive Verbindungen mit wenigstens zwei hydrophilen und wenigstens zwei hydrophoben Gruppen sind bisher nur auf der Basis von Diglycidylethern hergestellt worden (US 5 160 450, JP 01 304 033, JP 4 124 165). Diglycidylether gelten jedoch als toxikologisch bedenklich und sind recht teuer. Darüber hinaus wird für ihre Herstellung Epichlorhydrin verwendet, was zu großen Mengen an Reststoffen führt, so daß diese Verbindungen unter ökotoxikologischen wie auch ökonomischen Gesichtspunkten nicht mehr zeitgemäß sind.

Es bestand daher die Aufgabe, amphiphile Verbindungen aufzufinden, die wenigstens zwei hydrophile und wenigstens zwei hydrophobe Gruppen aufweisen, wobei die amphiphilen Verbindungen einen sehr hohen Wirkungsgrad, bezogen auf die Einsatzmenge, haben, und die darüber hinaus aus technisch leicht verfügbaren Rohstoffen ohne großen Anfall von unerwünschten Nebenprodukten hergestellt werden können.

Die Aufgabe wird erfindungsgemäß durch amphiphile Diamide, deren Grundkörper aus Dicarbonsäuren oder deren Ester und Aminen hergestellt werden können, gelöst. Die entsprechenden Di- oder Oligoamide können alkoxyliert werden. Diese nichtionischen amphiphilen Verbindungen werden in anionische amphiphile Verbindungen überführt, indem man z. B. die vorgenannten Verbindungen mit SO₃/Inertgas (oder Oleum, Chlorsulfonsäure oder Amidosulfonsäure), mit Polyphosphorsäure, mit einer Halogenessigsäure, mit einem Sulton, mit Maleinsäureanhydrid und Natriumhydrogensulfit, oder mit einem Taurin, umsetzt und jeweils anschließend neutralisiert.

Bei den erfindungsgemäßen anionischen amphiphilen Verbindungen handelt es sich daher um Verbindungen der allgemeinen Formel I 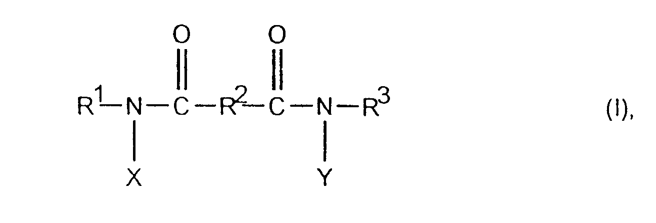 wobei R¹, R², R³, X und Y in der Formel I die im folgenden beschriebenen Bedeutungen haben:

R¹ und R³ stehen unabhängig voneinander für einen unverzweigten, gesättigten oder ungesättigten Kohlenwasserstoffrest mit 1 bis 22, vorzugsweise 7 bis 17, Kohlenstoffatomen.

Es seien als Substituenten R¹ und R³ im einzelnen die Reste Methyl, Ethyl, n-Propyl, n-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, n-Eicosyl, n-Henicosyl, n-Docosyl sowie die entsprechenden einfach, zweifach oder dreifach ungesättigten Reste genannt.

Der Spacer R² bedeutet
◆ unverzweigte oder verzweigte Alkylenketten der Formel II

   -CₐH₂ₐ- (II)

   mit a = 2 bis 18, vorzugsweise a = 3 bis 6;
◆ unverzweigte oder verzweigte Alkenylenketten der Formel III

   -C_{b}H_{2b}-CH=CH-C_{c}-H_{2c}- (III)

   mit b + c = 2 bis 16, wobei b und c jeweils größer als Null sind;
◆ unverzweigte oder verzweigte Alkinylenketten der Formel IV

   -C_{d}H_{2d}-C≡C-CₑH₂ₑ- (IV)

   mit d + e = 2 bis 16, wobei d und e jeweils größer als Null sind;
◆ Alicyclen gemäß der Formel V

   -C_{f}H_{2f}-cyclo C₆H₁₀-C_{g}H_{2g}- (V)

   mit f und g = je 1 bis 6
   oder gemäß der Formel VI

   -3(4),8(9)-di(methylen)-tricyclo[5.2.1.0^{2.6}]decan- (VI);
◆ unsubstituierte oder substituierte Aromaten gemäß der Formel VII

   -CₕH₂ₕ-C₆R₄-(CᵢH₂ᵢ-C₆R₄)_{j₁}-C_{j₂}H_{2j₂}- (VII)

   oder gemäß der Formel VIII

   -CₕH₂ₕ-C₁₀R₆-CⱼH₂ⱼ- (VIII)

   mit h, j, j₁ und j₂ = je 0 bis 8 und i = 1 bis 8 und
   mit R = unabhängig voneinander jeweils H oder C₁- bis C₄-Alkyl;
◆ eine Gruppe gemäß der Formel IX

   -CₖH₂ₖ-CₓR_{y}-Z-CₓR_{y}-CₗH₂ₗ- (IX)

   mit k und l = je 0 bis 8, x = 6 und y = 4 oder
   x = 10 und y = 6 oder x = 14 und y = 8, und
   Z = O, NH, NR¹, N-C(O)R¹, SO₂, wobei R¹ einen Kohlenwasserstoffrest mit 1 bis 22 Kohlenstoffatomen und R unabhängig voneinander jeweils H oder C₁- bis C₄-Alkyl bedeuten;
◆ eine Gruppe gemäß der Formel X

   -CₘH₂ₘ-(OCₙH₂ₙ)ₚ-C_{q}H_{2q}- (X)

   mit m = 1 bis 4, n = 2 bis 4, p = 1 bis 20, vorzugsweise p = 1 bis 6, und q = 1 bis 4,
   wobei auch gemischte Alkoxideinheiten auftreten können und dann die Reihenfolge der Alkoxideinheiten beliebig ist;
◆ eine Gruppe gemäß der Formel XI

   -CᵣH₂ᵣ(RNCₛH₂ₛ)ₜ-CᵤH₂ᵤ- (XI)

   oder gemäß der Formel XII

   -[CᵣH₂ᵣ[RN-C(O)-NR]ₜ-CᵤH₂ᵤ]_{w}- (XII)

   oder gemäß der Formel XIII

   -[CᵣH₂ᵣ[RNC(O)CᵥH₂ᵥC(O)NR]ₜ-CᵤH₂ᵤ]_{w}- (XIII)

   oder gemäß der Formel XIV

   -[CᵣH₂ᵣ[RN-C(O)-CH=CH-C(O)-NR]ₜ-CᵤH₂ᵤ]_{w}- (XIV)

   oder gemäß der Formel XV

   -[CᵣH₂ᵣ[RNC(O)CₓR_{y}C(O)NR]ₜ-CᵤH₂ᵤ]_{w}- (XV)

   mit r = 2 bis 4, s = 2 bis 4, t = 1 bis 20, vorzugsweise t = 1 bis 4, u = 2 bis 4 v =
   0 bis 12, w = 1 bis 6, x = 6 und y = 4 oder
   x = 10 und y = 6 oder
   x = 14 und y = 8
   mit R = unabhängig voneinander jeweils H oder C₁- bis C₄-Alkyl.
X oder Y stehen unabhängig voneinander für einen Substituenten der Formel XVI

-(C₂H₄O)_{α}(C₃H₆O)_{β}H (XVI)

mit
α = 0 bis 50, vorzugsweise α = 10 bis 30,
β = 0 bis 60, vorzugsweise β = 20 bis 40,
und α + β = 1 bis 100, vorzugsweise α + β = 10 bis 50,
der Formel XVIII

-CH₂[CHO(C₂H₄O)_{ε}(C₃H₆O)_{η}A]_{z}-CH₂-O(C₂H₄O)_{µ}(C₃H₆O)_{ρ}-A (XVIII)

mit
z = 3 bis 6, vorzugsweise z = 4
und ε bzw. µ = 0 bis 30, vorzugsweise ε bzw. µ = 0 bis 10,
und η bzw. ρ = 0 bis 30, vorzugsweise η bzw. ρ = 0 bis 10,
der Formel XVII

-(C₂H₄O)_{γ}(C₃H₆O)_{δ}-FR (XVII)

mit jeweils
γ = 0 bis 20, vorzugsweise γ = 0 bis 8,
δ = 0 bis 20, vorzugsweise δ = 0 bis 12,
und γ + δ = 1 bis 40, vorzugsweise γ + δ = 5 bis 20,
wobei FR für einen funktionellen Rest -CH₂-COOM, -SO₃M, -P(O)(OM)₂,

-O-C(O)-C₂H₃(SO₃M)-CO₂M'

oder -C₂H₄-SO₃M mit M, M' = Alkali, Ammonium-, Alkanolammonium oder ½ Erdalkali steht,
und wobei die Alkoxideinheiten bei den funktionellen Gruppen der Formeln XVI bis XVIII statistisch oder blockweise eingebunden sind und die Reihenfolge beliebig ist und wobei A für H oder FR steht.

Der Alkoxylierungsgrad ist jeweils ein Mittelwert und kann jeden beliebigen, auch nicht ganzzahligen Wert in den angegebenen Grenzen einnehmen.

Die erfindungsgemäßen amphiphilen Verbindungen zeichnen sich meist durch extrem niedrige kritische Micellbildungskonzentrationen (CMC) und sehr niedrige Oberflächen- und Grenzflächenspannungen (z. B. gegen Paraffin) aus, was auf ihre besondere Struktur - wenigstens zwei hydrophile Gruppen und wenigstens zwei hydrophobe Gruppen - zurückgeführt werden muß. Darüber hinaus weisen die meisten von ihnen ein recht hohes hydrophiles Suspendiervermögen auf, das etwa auf halbem Wege zwischen dem konventioneller Tenside und dem des Pentanatriumtripolyphosphats liegt. Einige dieser Verbindungen sind extrem schnelle Netzmittel.

Die amphiphilen Verbindungen gemäß dieser Erfindung eignen sich insbesondere als Emulgatoren, Demulgatoren, Detergenzien, Dispergatoren und Hydrotropica in Industrie und Haushalt, beispielsweise auf den Gebieten Metallbearbeitung, Erzgewinnung, Oberflächenveredelung, Waschen und Reinigen, Kosmetik, Medizin und Nahrungsmittelverarbeitung und -zubereitung.

Hierbei können sie mit allen gängigen anionischen, nichtionischen, kationischen und ampholytischen grenzflächenaktiven Substanzen kombiniert werden. Als Beispiele für nichtionische grenzflächenaktive Substanzen, die für eine Kombination eingesetzt werden können, seien genannt: Fettsäureglyceride, Fettsäurepolyglyceride, Fettsäureester, Ethoxylate höherer Alkohole, Polyoxyethylenfettsäureglyceride, Polyoxyethylenpropylenglykolfettsäureester, Polyoxyethylensorbitanfettsäureester, Polyoxyethylen-Rhizinusöl- oder gehärtete Rhizinusöl-Derivate, Polyoxyethylenlanolinderivate, Polyoxyethylenfettsäureamide, Polyoxyethylenalkylamine, Alkanolamine, Alkylaminoxide, Derivate von Eiweißhydrolysaten, Hydroxymischether, Alkylpolyglycoside und Alkylglucamide.

Als Beispiele für anionische grenzflächenaktive Substanzen, die für Kombinationen eingesetzt werden können, seien genannt: Seifen, Ethercarbonsäuren und deren Salze, Alkylsulfonate, α-Olefinsulfonate, Sulfonate höherer Fettsäureester, Alkoholsulfate, Alkoholethersulfate, Hydroxymischethersulfate, Salze von Phosphatestern, Tauride, Isethionate, lineare Alkylbenzolsulfonate, Cumolsulfonat, Alkylarylsulfonate, Sulfate der Polyoxyethylenfettsäureamide und Salze von Acylaminosäuren.

Als Beispiele für kationische gängige grenzflächenaktive Substanzen, die für Kombinationen eingesetzt werden können, seien genannt: Alkyltrimethylammoniumsalze, Dialkyldimethylammoniumsalze, Alkyldimethylbenzylammoniumsalze, Alkylpyridiniumsalze, Alkylisochinoliniumsalze, Benzethoniumchloride und kationische Acylaminosäurederivate.

Als Beispiele für ampholytische grenzflächenaktive Substanzen, die für Kombinationen eingesetzt werden können, seien genannt: Aminosäuren, Betaine, Sulfobetaine, Imidazolinderivate, Sojaöllipide und Lecithin.

Darüber hinaus können die erfindungsgemäßen amphiphilen Verbindungen auch für sich miteinander kombiniert werden.

Den erfindungsgemäßen amphiphilen Verbindungen können ebenfalls gängige Additive zugesetzt werden. Solche Additive werden speziell für eine Formulierung ausgewählt und umfassen üblicherweise anorganische Salze, wie Natriumchlorid und -sulfat, sowie Builder, Hydrotropica, UV-Absorber, Weichmacher, Chelatbildner, Viskositätsmodifizierer und Riechstoffe.

Die obengenannten Verbindungen lassen sich nach bekannten Methoden herstellen:

Die Dicarbonsäuren bzw. deren Methylester werden mit zwei Äquivalenten Amin bei erhöhten Temperaturen (80 bis 240 °C) optimal in Gegenwart eines Katalysators umgesetzt, wobei das entstehende Wasser oder Methanol unter Vakuum entfernt wird.

Handelt es sich um Dicarbonsäure-N,N'-dialkyldiamide werden sie bei Temperaturen von 130 bis 190 °C unter Druck in Gegenwart eines basischen Katalysators alkoxyliert. Bei Dicarbonsäuredialkylpolyhydroxydiamiden (Formel XVIII) ist dieser Schritt optional.

Die Produkte können anschließend mit SO₃/Inertgas (Oleum, Chorsulfonsäure oder Amidosulfonsäure) oder Polyphosphorsäure oder mit einer Halogenessigsäure, einem Sulton, mit Maleinsäureanhydrid und Natriumhydrogensulfit oder mit Isethionsäure umgesetzt und mit wäßrigen Alkali- oder Erdalkalihydroxiden oder Ammoniak oder Alkanolaminen neutralisiert werden.

## Patentansprüche

1. Anionische amphiphile Verbindungen der allgemeinen Formel 1 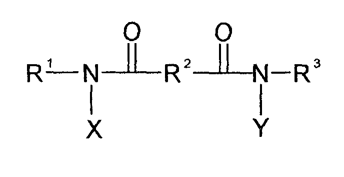 in der R¹ und R³ unabhängig voneinander für einen unverzweigten, gesättigten oder ungesättigten Kohlenwasserstoffrest mit 1 bis 22 Kohlenstoffatomen,
R² für eine unverzweigte oder verzweigte Alkylenkette der Formel II
-CₐH₂ₐ- (II)
mit a = 2 bis 18,
eine unverzweigte oder verzweigte Alkenylenkette der Formel III
-C_{b}H_{2b}-CH=CH-C_{c}H_{2c}- (III)
mit b + c = 2 bis 16, wobei b und c jeweils größer als Null sind,
eine unverzweigte oder verzweigte Alkinylenkette der Formel IV
-C_{d}H_{2d}-C≡C-CₑH₂ₑ- (IV)
mit d + e = 2 bis 16, wobei d und e jeweils größer als Null sind,
Alicyclen gemäß der Formel V
-C_{f}H_{2f}-cyclo C₆H₁₀-C_{g}H_{2g} - (V)
mit f und g = je 1 bis 6
oder gemäß der Formel VI
-3 (4), 8 (9) -di (methylen)-tricyclo [5.2.1.0^{2.6}] decan- (VI)
unsubstituierten oder substituierten Aromaten gemäß der Formel VII
-CₕH₂ₕ-C₆R₄- (CᵢH₂ᵢ-C₆R₄)ⱼ₁-Cⱼ₂H₂ⱼ₂ (VII)
oder gemäß der Formel VIII
-CₕH₂ₕ-C₁₀R₆-CⱼH₂ⱼ- (VIII)
mit h, j , j₁ und j₂ = je 0 bis 8 und i = 1 bis 8 und mit R = unabhängig voneinander jeweils H oder C₁- bis C₄-Alkyl,
eine Gruppe gemäß der Formel (IX)
-CₖH₂ₖ-CₓR_{y}-Z-CₓR_{y}CₗH₂ₗ- (IX)
mit k und 1 = je 0 bis 8, x = 6 und y = 4 oder
x = 10 und y = 6 oder x = 14 und y = 8, und
Z = 0, CO, NH, NR¹, N-C(O)R¹, SO₂,
wobei R¹ für einen Kohlenwasserstoffrest mit 1 bis 22 Kohlenstoffatomen und R unabhängig voneinander
jeweils für H oder C₁- bis C₄-Alkyl steht,
eine Gruppe gemäß der Formel X
-CₘH₂ₘ- (CₙH₂ₙO)ₚ-C_{q}H_{2q}- (X)
mit m = 1 bis 4, n = 2 bis 4, p = 1 bis 20, vorzugsweise p = 1 bis 6,
und q = 1 bis 4,
wobei auch gemischte Alkoxideinheiten auftreten können und dann die Reihenfolge der Alkoxideinheiten beliebig ist,
eine Gruppe gemäß der Formel XI
-CᵣH₂ᵣ(RNCₛH₂ₛ)ₜ-CᵤH₂ᵤ- (XI)
oder gemäß der Formel XII
-[CᵣH₂ᵣ[RN-C(O)-NR]ₜ-CᵤH₂ᵤ]_{w}- (XII)
oder gemäß der Formel XIII
-[CᵣH₂ᵣ[RNC(O)CᵥH₂ᵥC(O)NR]ₜ-CᵤH₂ᵤ]_{w}- (XIII)
oder gemäß der Formel XIV
-[CᵣH₂ᵣ[RN-C(O)-CH=CH-C(O)-NR]ₜ-CᵤH₂ᵤ]_{w}- (XVI)
oder gemäß der Formel XV
- [CᵣH₂ᵣ[RNC(O)CₓR_{y}C(O)NR]ₜ-CᵤH₂ᵤ]_{w}- (XV)
mit r = 2 bis 4, s = 2 bis 4, t = 1 bis 20, u = 2 bis 4,
v = 0 bis 12, w = 1 bis 6, x = 6 und y = 4 oder
x = 10 und y = 6 oder x = 14 und y = 8
mit R = unabhängig voneinander jeweils H oder C₁- bis C₄-Alkyl,
steht, und
X und Y unabhängig voneinander
für einen Substituenten der Formel XVI
-(C₂H₄O)_{α}(C₃H₆O)_{β}H (XVI)
mit α = 0 bis 50,
β = 0 bis 60,
und a + β = 1 bis 100,
oder für einen Substituenten der Formel XVII
-(C₂H₄O)_{γ}(C₃H₆O)_{δ}-FR (XVII)
mit jeweils γ = 0 bis 20,
δ = 0 bis 20,
und γ + δ= 1 bis 40,
wobei FR für einen funktionellen Rest -CH₂-COOM, -SO₃M, -P(O) (OM)₂, -O-C(O)-C₂H₃(SO₃M)-CO₂M'
oder -C₂H₄-SO₃M mit M, M' = Alkali, Ammonium-, Alkanolammonium oder 1/2 Erdalkali steht,
oder der Formel XVIII
-CH₂[CHO(C₂H₄O)_{ε} (C₃H₆O)_{η}A]_{z}-CH₂-O (C₂H₄O)_{µ}(C₃H₆O)_{ρ}-A (XVIII)
mit z = 3 bis 6,
und ε bzw. µ, = 0 bis 30,
und η bzw. ρ = 0 bis 30, und wobei A für H oder FR steht,
und wobei bei gemischten Alkoxiden die Alkoxideinheiten statistisch oder blockweise eingebunden sind und die Reihenfolge beliebig ist.

2. Verbindungen nach Anspruch 1, dadurch gekennzeichnet, daß der Spacer R² der Formel II entspricht und 2 bis 6 Kohlenstoffatome enthält.

3. Verwendung der Verbindungen nach einem der Ansprüche 1 oder 2 als Emulgator oder Demulgatoren.

4. Verwendung der Verbindungen nach einem der Ansprüche 1 oder 2 als Hilfsmittel bei der Metallbearbeitung, Erzgewinnung oder Oberflächenveredelung.

5. Verwendung der Verbindungen nach einem der Ansprüche 1 oder 2 als Textilhilfsmittel oder für das Reinigen und Waschen von Textilien.

6. Verwendung der Verbindungen nach einem der Ansprüche 1 oder 2 für das Reinigen von harten Oberflächen.

7. Verwendung der Verbindungen nach einem der Ansprüche 1 oder 2 für das Reinigen und Waschen von Haut und Haar.

## Claims

1. Amphiphilic anionic compounds of the general formula I 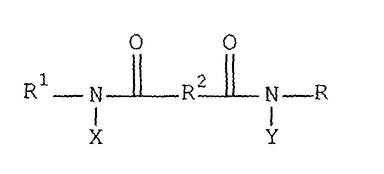 where **R**^{**1**} and **R**^{**3**}**,** independently of one another, are an unbranched, saturated or unsaturated hydrocarbon radical having 1 to 22 carbon atoms,
**R**^{**2**} is an unbranched or branched alkylene chain of the formula II
-CₐH₂ₐ- **(II)**
where **a** = 2 to 18;
an unbranched or branched alkenylene chain of the formula III
-C_{b}H_{2b}-CH=CH-C_{c}H_{2c}- **(III)**
where **b** + **c** = 2 to 16, each **b** and **c** being greater than zero;
an unbranched or branched alkynylene chain of the formula IV
-C_{d}H_{2d}-C≡C-CₑH₂ₑ- **(IV)**
where **d** + **e** = 2 to 16, each **d** and **e** being greater than zero;
alicyclic compounds of the formula V
-C_{f}H_{2f}-cycloC₆H₁₀-C_{g}H_{2g}- **(V)**
where **f** and **g** = 1 to 6 each,
or of the formula VI
-3(4),8(9)-di(methylene)-tricyclo[5.2.1.0^{2.6}] decane-; **(VI)**
unsubstituted or substituted aromatics of the formula VII
-CₕH₂ₕ-C₆R₄-(CᵢH₂ᵢ-C₆R₄)_{j₁}-C_{j₂}H_{2j₂}- **(VII)**
or of the formula VIII
-CₕH₂ₕ-C₁₀R₆-CⱼH₂ⱼ- **(VIII)**
where each **h, j, j**_{**1**}**,** and **j**_{**2**} = 0 to 8, and **i** = 1 to 8, and each R, independently of one another, is equal to H or C₁- to C₄-alkyl;
a group of the formula IX
-CₖH₂ₖ-CₓR_{y}-Z-CₓR_{y}-CₗH₂ₗ- **(IX)**
where each **k** and **l** = 0 to 8, **x** = 6 and **y** = 4, or **x** = 10 and **y** = 6, or **x** = 14 and **y** = 8, and
**Z** = 0, CO, NH, NR¹, N-C(O)R¹, SO₂,
where **R**^{**1**} is a hydrocarbon radical having from 1 to 22 carbon atoms and each **R**, independently of one another, is H or C₁- to C₄-alkyl;
a group of the formula X
-CₘH₂ₘ-(CₙH₂ₙO)ₚ-C_{q}H_{2q}- **(X)**
where **m** = 1 to 4, **n** = 2 to 4, **p** = 1 to 20, preferably **p** = 1 to 6,
and **q** = 1 to 4,
wherein also mixed alkoxide units may be present, and if so, the sequence of said alkoxide units is optional;
a group of the formula XI
-CᵣH₂ᵣ(RNCₛH₂ₛ)ₜ-CᵤH₂ᵤ- **(XI)**
or of the formula XII
-[CᵣH₂ᵣ[RN-C(O)-NR]ₜ-CᵤH₂ᵤ]_{w}- **(XII)**
or of the formula XIII
-[CᵣH₂ᵣ[RNC(O)CᵥH₂ᵥC(O)NR]ₜ-CᵤH₂ᵤ]_{w}- **(XIII)**
or of the formula XIV
- [CᵣH₂ᵣ[RN-C(O)-CH=CH-C (O)-NR] ₜ-CᵤH₂ᵤ] w- **(XIV)**
or of the formula XV
-[CᵣH₂ᵣ(RNC(O)CₓR_{y}C(O)NR]ₜ-CᵤH₂ᵤ]_{w}- **(XV)**
where **r** = 2 to 4, **s** = 2 to 4, **t** = 1 to 20,
**u** = 2 to 4, **v** = 0 to 12, **w** = 1 to 6,
**x** = 6 and **y** = 4, or
**x** = 10 and **y** = 6, or
**x** = 14 and **y** = 8,
and each **R**, independently of one another, is equal to H or C₁- to C₄-alkyl, and
**X** and **Y** are independently of one another a substituent of the formula XVI
-(C₂H₄O)_{α}(C₃H₆O)_{β}H **(XVI)**
where α = 0 to 50,
β = 0 to 60,
and α + β = 1 to 100,
or a substituent of the formula XVII
-(C₂H₄O)γ(C₃H₆O)_{δ}-FR **(XVII)**
where each γ = 0 to 20,
δ = 0 to 20,
and γ + δ = 1 to 40,
and **FR** is a functional radical -CH₂-COOM, -SO₃M, -P(O) (OM)₂, -O-C(O)-C₂H₃(SO₃M)-CO₂M',
or -C₂H₄-SO₃M, where M, M' = alkali metal, ammonium, alkanol ammonium, or ½ alkaline earth metal ion,
or of the formula XVIII
-CH₂[CHO(C₂H₄O)_{ε}(C₃H₆O)_{η}A]_{z}-CH₂-O(C₂H₄O)_{µ} (C₃H₆O)_{ρ}-A **(XVIII)**
where **z** = 3 to 6,
and ε or µ = 0 to 30,
and η or ρ = 0 to 30,
and A represents H or FR,
and the alkoxide units of mixed alkoxides are incorporated randomly or blockwise and the sequence is optional.

2. The compounds according to claim 1,
**characterized in that** the spacer R² corresponds to the formula II and comprises 2 to 6 carbon atoms.

3. Use of the compounds according to claim 1 or 2 as emulsifiers or demulsifiers.

4. Use of the compounds according to claim 1 or 2 as auxiliaries in metal working, ore mining, or surface finishing.

5. Use of the compounds according to claim 1 or 2 as textile auxiliaries or for cleaning and washing textiles.

6. Use of the compounds according to claim 1 or 2 for cleaning hard surfaces.

7. Use of the compounds according to claim 1 or 2 for cleaning and washing skin and hair.

## Revendications

1. Composés amphiphiles anioniques de formule générale I 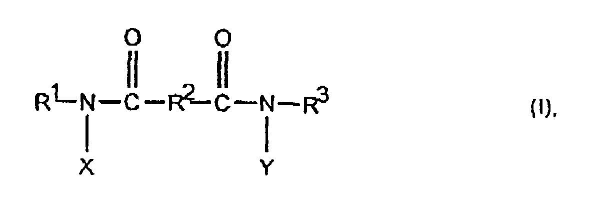 dans laquelle R¹ et R³ représentent, indépendamment l'un de l'autre, un radical hydrocarboné linéaire, saturé ou insaturé comprenant 1 à 22 atomes de carbone
R² représente
une chaîne alkylène linéaire ou ramifiée de formule II
-CₐH₂ₐ- (II)
avec a = 2 à 18,
une chaîne alcénylène linéaire ou ramifiée de formule III
-C_{b}H_{2b}-CH=CH-C_{c}H_{2c}- (III)
avec b + c = 2 à 16, b et c étant à chaque fois supérieurs à 0,
une chaîne alcynylène linéaire ou ramifiée de formule IV
-C_{d}H_{2d}-C≡C-CₑH₂ₑ- (IV)
avec d + e = 2 à 16, d et e étant à chaque fois supérieurs à 0,
des alicycles selon la formule V
-C_{f}H_{2f}-cyclo C₆H₁₀-C_{g}H_{2g}- (V)
avec f et g = chacun 1 à 6,
ou selon la formule VI
-3(4),8(9)-di(méthylène)tricyclo[5.2.1.0^{2.6}]décane- (VI)
des aromates non substitués ou substitués selon la formule VII
-CₕH₂ₕ-C₆R₄-(CᵢH₂ᵢ-C₆R₄)ⱼ₁-Cⱼ₂H₂ⱼ₂- (VII)
ou selon la formule VIII
-CₕH₂ₕ-C₁₀R₆-CⱼH₂ⱼ- (VIII)
avec h, j, j₁ et j₂ = chacun 0 à 8 et i = 1 à 8
avec R = indépendamment l'un de l'autre à chaque fois H ou alkyle en C₁ à C₄,
un groupement selon la formule IX
-CₖH₂ₖ-CₓR_{y}-Z-CₓR_{y}-CₗH₂ₗ- (IX)
avec k et l = chacun 0 à 8, x = 6 et y = 4 ou
x = 10 et y = 6 ou x = 14 et y = 8 et
Z = O, CO, NH, NR¹, N-C(O)R¹, SO₂, où R¹ signifie un radical hydrocarboné comprenant 1 à 22 atomes de carbone et R signifie, indépendamment l'un de l'autre, à chaque fois H ou alkyle en C₁ à C₄,
un groupement selon la formule X
-CₘH₂ₘ-(OCₙH₂ₙ)ₚ-C_{q}H_{2q}- (X)
avec m = 1 à 4, n = 2 à 4, p = 1 à 20, de préférence p = 1 à 6, et q = 1 à 4,
des unités mixtes alkoxyde pouvant également exister et l'ordre des unités alkoxyde étant quelconque,
un groupement selon la formule XI
-CᵣH₂ᵣ(RNCₛH₂ₛ)ₜ-CᵤH₂ᵤ- (XI)
ou selon la formule XII
- [CᵣH₂ᵣ[RN-C(O)-NR]ₜ-CᵤH₂ᵤ]_{w}- (XII)
ou selon la formule XIII
- [CᵣH₂ᵣ[RNC(O)CᵥH₂ᵥC(O)NR]ₜ-CᵤH₂ᵤ]_{w}- (XIII)
ou selon la formule XIV
- [CᵣH₂ᵣ[RN-C(O)-CH=CH-C(O)-NR]ₜ-CᵤH₂ᵤ)_{w}- (XIV)
ou selon la formule XV
- [CᵣH₂ᵣ [RNC (O) CₓR_{y}C (O) NR]ₜ-CᵤH₂ᵤ]_{w}- (XV)
avec r = 2 à 4, s = 2 à 4, t = 1 à 20, u = 2 à 4, v = 0 à 12, w = 1 à 6, x = 6 et y = 4 ou
x = 10 et y = 6 ou
x = 14 et y = 8
avec R = indépendamment l'un de l'autre à chaque fois H ou alkyle en C₁ à C₄, et
X ou Y représentent, indépendamment l'un de l'autre, un substituant de formule XVI
- (C₂H₄0)_{α}(C₃H₆0)_{β}H (XVI)
avec α = 0 à 50,
β = 0 à 60,
et a+β = 1 à 100,
ou un substituant de formule XVII
-(C₂H₄O)_{γ}(C₃H₆O)_{δ}-FR (XVII)
avec à chaque fois γ = 0 à 20,
δ = 0 à 20,
γ+δ = 1 à 40,
FR représentant un radical fonctionnel -CH₂-COOM, -SO₃M, -P(O)(OM)₂, -O-C(O)-C₂H₃(SO₃M)-CO₂M' ou -C₂H₄SO₃M, avec M, M' = alcali, ammonium, alcanolammonium ou 1/2 alcalino-terreux,
ou un substituant de formule XVIII
-CH₂[CHO(C₂H₄O_{ε}(C₃H₆O)_{η}A]_{z}-CH₂-O(C₂H₄O)_{µ}(C₃H₆O)_{ρ}-A (XVIII)
avec z = 3 à 6,
et ε ou selon le cas µ = 0 à 30,
et η ou selon le cas ρ = 0 à 30,
et A représentant H ou FR,
et, pour des alkoxydes mixtes, les unités alkoxyde étant insérées statistiquement ou en blocs et l'ordre étant quelconque.

2. Composés selon la revendication 1, caractérisés en ce que l'espaceur R² correspond à la formule II et contient 2 à 6 atomes de carbone.

3. Utilisation des composés selon l'une quelconque des revendications 1 ou 2 comme émulsifiant ou désémulsifiant.

4. Utilisation des composés selon l'une quelconque des revendications 1 ou 2 comme adjuvant lors du traitement de métaux, de l'extraction de minerais ou du finissage de surfaces.

5. Utilisation des composés selon l'une quelconque des revendications 1 ou 2 comme adjuvant pour textiles ou pour le lavage et le nettoyage de textiles.

6. Utilisation des composés selon l'une quelconque des revendications 1 ou 2 pour le nettoyage de surfaces dures.

7. Utilisation des composés selon l'une quelconque des revendications 1 ou 2 pour le nettoyage et le lavage de la peau et des cheveux.
